# EUROPÄISCHE PATENTANMELDUNG

(11) **EP 1 616 497 A2**
(43) Veröffentlichungstag der Anmeldung: **18.01.2006**
(21) Anmeldenummer: 05013865.0
(22) Anmeldetag: 28.06.2005
(51) Int. Cl.: A45C 11/00

(54) **Erste-Hilfe-Materialbehältnis**

(30) Priorität: 12.07.2004 DE 102004033803
(71) Anmelder: Paul Hartmann AG, 89522 Heidenheim (DE)
(72) Erfinder: Fuchs, Sabine, Dr., 89518 Heidenheim (DE); Mangold, Rainer, Dr., 89542 Herbrechtingen (DE); Kelm, Olga, 89522 Heidenheim (DE)
(74) Vertreter: Langöhrig, Angelika Beate

(57) **Zusammenfassung**

Die Erfindung betrifft ein Erste-Hilfe-Materialbehältnis umfassend eine erstes Kompartement (10) zur Aufnahme von Erste-Hilfe-Material sowie mindestens ein weiteres Kompartement (12, 44), wobei mindestens ein Kompartement (10, 12, 44) öffen- und schließbar ist, dadurch gekennzeichnet, dass mindestens eines der weiteren Kompartements (12) zur Aufnahme einer Schutzbekleidung dient und mindestens eine der Wandungen (26) des Kompartements (12) zur Aufnahme der Schutzbekleidung eine Sichtseite bildet und so gestaltet ist, dass die Schutzbekleidung von außen sichtbar ist.

## Beschreibung

Die Erfindung betrifft ein Erste-Hilfe-Materialbehältnis umfassend ein erstes Kompartement zur Aufnahme von Erste-Hilfe-Material sowie mindestens ein weiteres Kompartement, wobei mindestens ein Kompartement öffen- und schließbar ist.

Üblicherweise wird das Erste-Hilfe-Material, insbesondere für Kraftfahrzeuge, entweder in Hartschalenkunststoffboxen oder in weichen kissenähnlichen Verpackungen untergebracht.

Weiterhin müssen in der Regel noch ein oder zwei Warndreiecke mitgeführt werden. Schließlich ist es wünschenswert, nicht nur die Unfallstelle durch Aufstellen eines Warndreiecks zu sichern, sondern auch den in den Unfall involvierten Personen sowie zu Hilfe eilenden Rettern eine größtmögliche Sicherheit durch gute Kennzeichnung zu gewähren.

Problematisch ist nun, dass in aller Regel sämtliche hierfür notwendigen Gegenstände und Materialien entweder separat aufbewahrt werden müssen oder in einer einzigen Hülle, nämlich dem Erste-Hilfe-Materialbehältnis zusammen aufbewahrt werden, was jedoch die Übersichtlichkeit einschränkt, so dass es in vielen Fällen dazu kommen wird, dass beispielsweise Schutzbekleidung, aber auch Rettungsdecken etc. durch die Retter nicht aufgefunden bzw. aufgrund der Vielzahl der zusammen verstauten Sachen nicht erkannt werden.

In diesem Fall können jedoch zusätzliche Sicherheits- und Rettungsmittel nicht genutzt werden und erfüllen somit nicht ihren eigentlichen Zweck.

Um dies zu verbessern, ist beispielsweise aus der DE 201 14 150 Ul ein Behältnis zur Aufnahme von Verbandstoffen und einem Euro-Warndreieck bekannt, wobei zwei voneinander getrennte Fächer vorgesehen sind, in denen einmal Verbandstoffe und einmal ein Euro-Warndreieck untergebracht ist.

Ausgehend hiervon ist es nun Aufgabe der Erfindung, ein Erste-Hilfe-Materialbehältnis bereitzustellen, dass neben einem Kompartement für das Erste-Hilfe-Material ein weiteres separates Kompartement für die Aufnahme einer Schutzbekleidung vorgesehen ist, wobei eine gute Übersichtlichkeit und Lagerbarkeit sowie eine leichte Auffindbarkeit der Schutzbekleidung gegeben sein soll.

Die Erfindung löst diese Aufgabe durch ein Erste-Hilfe-Materialbehältnis der gattungsgemäßen Art, bei dem mindestens eines der weiteren Kompartements zur Aufnahme einer Schutzbekleidung dient und mindestens eine der Wandungen des Kompartements zur Aufnahme der Schutzbekleidung eine Sichtseite bildet und so gestaltet ist, dass die Schutzbekleidung von außen sichtbar ist. Die Sichtseite ist dabei eine Außenseite des Behältnisses. Unter einem Kompartement soll erfindungsgemäß eine Aufnahme oder ein Fach im weitesten Sinne verstanden werden.

Auf diese Weise wird erreicht, dass auch in der Hektik eines Notfalls, insbesondere eines Verkehrsunfalls, die am Unfall Beteiligten bzw. eine zu Hilfe eilende Person, nach Auffindung des Verbandmaterial- bzw. Erste-Hilfe-Materialbehältnisses unmittelbar, auch eine Schutzbekleidung, insbesondere eine Warnweste, in leuchtenden und insbesondere auch reflektierenden Farben zusammen mit dem Verbandmaterial auffindet und anlegen kann und dass diese dann einer häufigeren Benutzung zugeführt wird und auf diese Weise die Sicherheit sowohl der Betroffenen als auch von helfenden Personen erhöht werden kann, durch Verbesserung deren Sichtbarkeit.

Nach einer ersten und weiteren Ausgestaltung kann vorgesehen sein, dass ein drittes und/oder viertes Kompartement zur Aufnahme eines oder mehrerer Warndreiecke vorgesehen ist. So ist insbesondere in manchen Ländern vorgeschrieben, dass nicht lediglich ein, sondern zwei Warndreiecke mitgeführt werden müssen. Durch die Anordnung eines weiteren oder mehrerer Kompartements derart, dass Erste-Hilfe-Material, Warndreieck bzw. Warndreiecke und Schutzbekleidung zusammen angeordnet sind, wird die Auffindbarkeit sämtlicher Utensilien für Beteiligte am Unfall, insbesondere auch in fremden Fahrzeugen, aber auch von zu Hilfe eilenden Rettern deutlich erleichtert.

Aufgrund der Tatsache, dass die Schutzbekleidung gut sichtbar an dem Behältnis angebracht ist, wird in aller Regel die Schutzbekleidung zu allererst aus ihrem Kompartement entnommen und angelegt werden. Wird dann beispielsweise als nächstes erste Hilfe geleistet, oder aber auch zunächst das Warndreieck aufgestellt, liegt hierbei bereits ein höherer Schutz der helfenden Person gegen Verletzungen z. B. durch nachfolgende Kraftfahrzeuge vor, da die Sichtbarkeit deutlich erhöht ist.

Dabei kann vorgesehen sein, dass z. B. zwei Warndreiecke in jeweils separaten Kompartements untergebracht sind, so dass auch hier die Entnehmbarkeit der Warndreiecke verbessert wird und beispielsweise ein Verkeilen der Warndreiecke ineinander vermieden werden kann.

Eine weitere Umverpackung der Warndreiecke, wie sie zumeist in einem Kunststoffköcher vorgesehen ist, ist dann nicht weiter notwendig. Es kann jedoch alternativ vorgesehen sein, jedes der Warndreiecke innerhalb seines Kompartements oder Fachs gesondert unterzuverpacken.

Es kann vorgesehen sein, dass das Behältnis zumindest teilweise aus einem nicht formstabilen, insbesondere textilen Material besteht, insbesondere kann es sich um ein wasserabweisendes bzw. wasserdichtes Material handeln, insbesondere ein Polyester mit einer Polyurethan- oder PVC-Beschichtung. Hierdurch wird die Verstaubarkeit insgesamt verbessert und das Behältnis kann für weitere Aufgaben, insbesondere auch als Kopfkissen zum Unterlegen unter eine verletzte Person, verwandt werden, sofern die benötigten Utensilien herausgenommen sind oder weitere Verbandmaterialbehältnisse zur Verfügung stehen.

Insbesondere können auch weitere Utensilien in separaten Kompartements, wie beispielsweise Rettungsdecken, Dokumente über den Fahrzeuginhaber für die medizinische Versorgung, etc. untergebracht sein.

Des Weiteren kann vorgesehen sein, dass an einem oder mehreren Kompartements insbesondere Velcro-Elemente angebracht sind, mit denen das Behältnis beispielsweise an einem Veloursbelag oder an zugeordneten korrespondierenden Velcro-Elementen in z. B. einem Kraftfahrzeug festgelegt ist, um ein Verrutschen und damit auch Abhandenkommen des Behältnisses zu verhindern, und es sicher an einem Ort zu verankern.

Nach einer weiteren Ausführungsform kann vorgesehen sein, dass die Kompartements (Fächer) zumindest zum Teil unabhängig voneinander öffen- und schließbar sind. Alternativ kann vorgesehen sein, dass beispielsweise das Kompartement für die Schutzbekleidung an einer oder mehreren, insbesondere auch zwei z. B. gegenüberliegenden Seiten offen gestaltet sein kann, z. B. ähnlich eines tunnelartigen Fachs oder einer aufgesetzten Tasche, wobei die offene Seite beispielsweise mit einem Gummizug gesichert werden kann, so das die Schutzbekleidung nur unter elastischer Verformung des Gummizugs aus dem Kompartement entnommen werden kann.

Weiterhin kann auch vorgesehen sein, dass beispielsweise zwei Kompartements miteinander gekoppelt sein können und sich gemeinsam öffnen und schließen lassen.

Als Mittel zum Öffnen und Schließen können insbesondere Reißverschlüsse vorgesehen sein.

Nach einer bevorzugten Ausführungsform kann vorgesehen sein, dass die Kompartements im Wesentlichen eine rechteckige Grundfläche aufweisen und jeweils einen Boden, einen Deckel und vier Seitenwände besitzen. Alternativ kann insbesondere bei einer taschenartigen Gestaltung für die Schutzbekleidung lediglich eine Vorsehung von drei Seitenwänden gegeben sein, wobei insbesondere die an die fehlende Seitenwand angrenzenden Seitenwände eine von einer rechteckigen Fläche abweichende, insbesondere dreieckige oder trapezförmige Form aufweisen können, die sich in Richtung auf die fehlende Seitenfläche verjüngen. Weiter alternativ kann das Kompartement für die Schutzbekleidung lediglich aus einem Boden und einem Deckel bestehen, die unmittelbar miteinander verbunden sind.

Die Reißverschlüsse können insbesondere so angebracht sein, dass sie im Bereich der Seitenwände oder zwischen den Seitenwänden und dem Deckel bzw. Boden eines Kompartements angeordnet sind und insbesondere umlaufend oder drei Seiten überspannend vorgesehen sind, wobei in diesem Fall dann die verbleibende Seite als Scharnier beim Öffnen wirkt.

Insbesondere beim Kompartement für das Erste-Hilfe-Material kann vorgesehen sein, dass das Erste-Hilfe-Material mit insbesondere dem Boden und/oder dem Deckel des Kompartements lösbar verbunden ist, so dass beim Aufklappen die Erste-Hilfe-Materialien mit dem Boden und/oder dem Deckel mitverschwenkt werden können bzw. am Boden fixiert sind, um eine übersichtliche Darbietung des Verbandmaterials und sonstiger Erste-Hilfe-Gegenstände sicherzustellen.

Nach einer ersten bevorzugten Ausführungsform kann vorgesehen sein, dass die Sichtseite durch einen Deckel bzw. eine Bodenseite, je nach Betrachtung, eines Kompartements gebildet ist.

Es kann dabei vorgesehen sein, dass die Sichtseite aus einem Netzmaterial besteht. Bei dem Netzmaterial kann es sich um ein Gestrick, ein Gewirk oder ein Gewebe handeln.

Besonders bevorzugt kann das Netzmaterial aus transluzenten, vorzugsweise annähernd transparenten Nylonfäden bestehen, da auf diese Weise eine fast 100%-ige durchscheinende Wirkung erreicht werden kann. Das Netz kann jedoch auch undurchsichtige, insbesondere farbige Fäden umfassen. Alternativ kann das Netzmaterial aus Polyesterfäden gebildet sein, wobei die größte Erstreckung einer jeden Netzöffnung zwischen 1 und 6 mm, vorzugsweise 2 und 5 mm und insbesondere 2 und 4 mm betragen kann. Die Netzöffnungen können vorzugsweise eine sechseckige, wabenartige Form aufweisen.

Darüber hinaus kann vorgesehen sein, dass der vom Netz abgedeckte Teil der Sichtseite zwischen 30 und 70 %, vorzugsweise zwischen 35 und 50 % und vorzugsweise zwischen 40 und 45 % Abdeckung im Vergleich zur freigelassenen Fläche liegt.

Weiterhin kann vorgesehen sein, dass ein Gewirk eingesetzt wird mit einer Lochgröße von ca. 5 bis 20 mm², vorzugsweise zwischen 9 und 12 mm², da so auf der einen Seite ein ausreichender Schutz der Schutzbekleidung in ihrem Kompartement erreicht wird, auf der anderen Seite jedoch eine gute Sichtbarkeit erzielt werden kann.

Die Bestimmung der Lochgröße und der abgedeckten bzw. freien Fläche erfolgte mittels eines Zeiss Stemi SV 11, einem Stereomikroskop mit 10-facher Vergrößerung und bei Auflicht sowie einer Fujix Digitalkamera HC-300Z. Zur Auswertung wurde eine Bildbearbeitungssoftware "Image Access 3.1.0" der Firma Imagic Bildverabeitung AG verwendet.

Schließlich kann das Netz elastifiziert sein, vorzugsweise elastische Fäden umfassen.

Alternativ kann die Sichtseite auch durch ein durchsichtiges Folienmaterial gebildet sein.

Des Weiteren kann vorgesehen sein, dass mindestens zwei Kompartements über ihre Böden miteinander verbunden sind, insbesondere beide Kompartements einen gemeinsamen Boden aufweisen. Auf diese Weise wird eine besonders einfache Gestaltung und Zugänglichkeit erreicht. Alternativ können jedoch auch separate Böden vorgesehen sein und es kann auch vorgesehen sein, dass die Kompartements vollständig voneinander separiert werden können.

Die Verbindung zweier Kompartements kann dabei entweder über Klettelemente sowie andere form- und kraftschlüssige Elemente, wie beispielsweise Druckknöpfe etc. sowie Reißverschlüsse vorgesehen sein.

Besonders bevorzugt ist, wenn die Sichtseite hinsichtlich ihrer Abmessungen mit einer Boden- und/oder Deckelfläche korrespondiert, d. h. vorzugsweise die Boden- oder Deckelfläche ganz die Sichtseite bildet. Alternativ können jedoch auch Ausgestaltungen vorgesehen sein, bei denen die Boden- oder Deckelfläche nur teilweise als Sichtseite ausgebildet ist. Hierbei gilt auch, dass, je größer die Sichtseite ist, ein Erkennen der Schutzbekleidung auch in hektischen Rettungssituationen besser gewährleistet werden kann.

Schließlich kann vorgesehen sein, dass die Kompartements an die Form und Größe der aufzunehmenden Gegenstände angepasst sind. Dabei kann z. B. vorgesehen sein, dass das Kompartement für das Verbandmaterial andere Abmessungen aufweist als das Kompartement beispielsweise für ein Warndreieck oder für eine Schutzbekleidung oder Rettungsdecke.

Nach einer alternativen Ausgestaltung kann vorgesehen sein, dass mindestens zwei Kompartements die gleiche Grundfläche aufweisen. Auf diese Weise wird eine sehr kompakte Gestaltung erreicht, die insbesondere eine gute Verstaubarkeit gewährleistet.

Weitere Vorteile und Merkmale der Erfindung ergeben sich aus der nachfolgenden Beschreibung von Ausführungsbeispielen. Dabei zeigen in schematischer Darstellung:

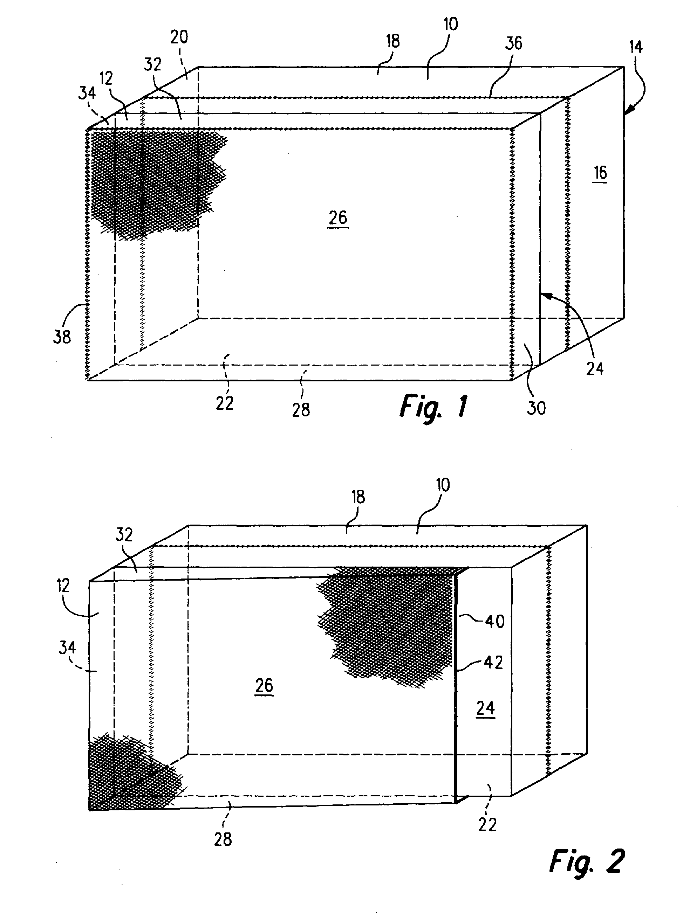

- Figuren 1 bis 5: verschiedene Ausgestaltungen eines erfindungsgemäßen Erste-Hilfe-Materialbehältnisses.

Figur 1 zeigt eine erste Ausgestaltung eines erfindungsgemäßen Erste-Hilfe-Materialbehältnisses umfassend ein erstes Kompartement 10 sowie ein zweites Kompartement 12, wobei das erste Kompartement eine rechteckige Grundfläche besitzt, und die Grundfläche des zweiten Kompartements mit der Grundfläche des ersten Kompartements übereinstimmt. Dabei weist das erste Kompartement 10 einen Deckel 14 auf sowie vier Seitenwände 16, 18, 20 und 22 sowie einen Boden 24, wobei der Boden 24 mit dem Boden des zweiten Kompartements 12 zusammenfällt. Das zweite Kompartement 12 umfasst ebenfalls einen Deckel 26 sowie vier Seitenwände 28, 30, 32 und 34. D. h. beide Kompartements 10, 12 besitzen eine quaderförmige Form und weisen die gleichen Abmessungen auf.

Beide Kompartements 10 und 12 sind wiederverschließbar gestaltet und weisen hierzu einen Reißverschluss 36 für das erste Kompartement 10 sowie einen Reißverschluss 38 für das zweite Kompartement 12 auf. Der Reißverschluss 36 des ersten Kompartements ist dabei an drei Seiten, nämlich 16, 18 und 20, angebracht und erstreckt sich durchgehend über diese drei Seiten, so dass die Seite 22 als Scharnier beim Aufklappen des Deckels 14 wirkt. Der Reißverschluss 38 des zweiten Kompartements ist dabei an der Kante zwischen Deckel 26 und den Seitenwänden 30, 32 und 34 angebracht, so dass die Kante zwischen dem Deckel 26 und der ersten Seite 28 ebenfalls eine Scharnierfunktion bekommt.

Das zweite Kompartement 12 ist dabei hinsichtlich seines Deckels 26 aus einem Netzmaterial gestaltet, wobei das Netzmaterial aus Polyesterfäden gewirkt ist mit einem Flächengewicht von 128 g/m² und die Lochgröße des Netzes 10,6 mm² beträgt. Die Lochöffnung besitzt eine größte Länge von 2,6 mm, was bedeutet, dass die durch das Netz freigelassene Fläche 58 % im Hinblick zu der durch das Netz abgedeckten Fläche von 42 % ausmacht. Die Netzöffnungen sind wabenartig ausgeführt.

Durch das an der Sichtseite 26, die hier die gesamte Deckelfläche umfasst, angeordnete Netzmaterial kann eine darunter angeordnete Schutzbekleidung, hier eine Warnweste nach der entsprechenden DIN EN 471, durch einen Benutzer des Erste-Hilfe-Behältnisses schnell ersehen werden, wodurch die Wahrscheinlichkeit des Erkennens des Mitführens der Warnweste erleichtert wird und damit auch die Wahrscheinlichkeit für die Benutzung der Warnweste erhöht wird. Auf diese Weise kann zur Sicherheit der Helfenden bzw. der Beteiligten in einem Unfallgeschehen beigetragen werden. Durch die Lochstruktur wird darüber hinaus eine Belüftung der Weste sichergestellt. Im ersten Kompartement 10 ist darüber hinaus Erste-Hilfe-Material sicher und staubgeschützt sowie geschützt gegen Nässe und Verschmutzung aufbewahrt. Dabei kann vorgesehen sein, dass das Erste-Hilfe-Material steril verpackt ist und die Behältnisse im Inneren des Kompartements 10 festgelegt sind, so dass das Aufklappen erleichtert wird und danach das Erste-Hilfe-Material gut dargeboten ist. Insbesondere können zwei Erste-Hilfe-Materialpäckchen vorgesehen sein, wobei das eine mit dem Boden 24 und das andere mit dem Deckel 14 des ersten Kompartements 10 verbunden ist.

Dabei kann vorgesehen sein, dass das Erste-Hilfe-Behältnis in seiner Gesamtheit aus einem textilen Material besteht, so dass das Behältnis sich in einem gewissen Maße formverändern lässt und dadurch eine gute Verstaubarkeit besitzt. Das Material ist dabei wasserabweisend.

Figur 2 zeigt nun eine alternative Ausgestaltung, bei der das erste Kompartement, wie in Figur 1 dargestellt, gestaltet ist, wobei jedoch in Figur 2, abweichend zu Figur 1, die Aufteilung des Kompartements in einen Bereich des Deckels und einen Bereich des Bodens durch den Reißverschluss hier gleichmäßig angeordnet ist, wobei in Figur 1 eine Verteilung 1/3 zu 2/3 zugunsten des Deckels vorgenommen ist.

Darüber hinaus ist ein zweites Kompartement 12 vorgesehen, das hier taschenartig gestaltet ist und ebenfalls einen Boden aufweist, der mit dem Boden 24 des ersten Kompartements 10 zusammenfällt. Darüber hinaus sind drei Seitenflächen 28, 32 und 34 vorgesehen sowie eine offene Seite 40. Die Seitenflächen 28 und 32 sind hier nicht rechteckig von der Grundfläche, sondern trapezförmig und verjüngen sich in Richtung auf die offene Seite 40. Die Erstreckung der Seiten 28 und 32 ist hierbei in ihrer Längsrichtung geringer als die Erstreckung der Seiten 18 und 22 des ersten Kompartements 10. Die Deckelfläche 26 des zweiten Kompartements 12 ist, ebenso wie bei Figur 1, aus einem Netzmaterial der vorstehend beschriebenen Art gestaltet.

Die Kante zwischen der offenen Seite 40 und der Deckelfläche 26 ist hierbei mit einem Gummiband 42 versehen, so dass die Kante in Richtung auf die Bodenfläche 24 des ersten Kompartements 10 vorgespannt ist. Unter Überwindung dieser Vorspannung kann dann eine Schutzbekleidung, insbesondere eine Warnweste, in das zweite Kompartement 12 eingeführt werden bzw. hieraus entnommen werden. Eine derartige Gestaltung bietet den Vorteil, dass auf die Schutzbekleidung schnell zugegriffen werden kann.

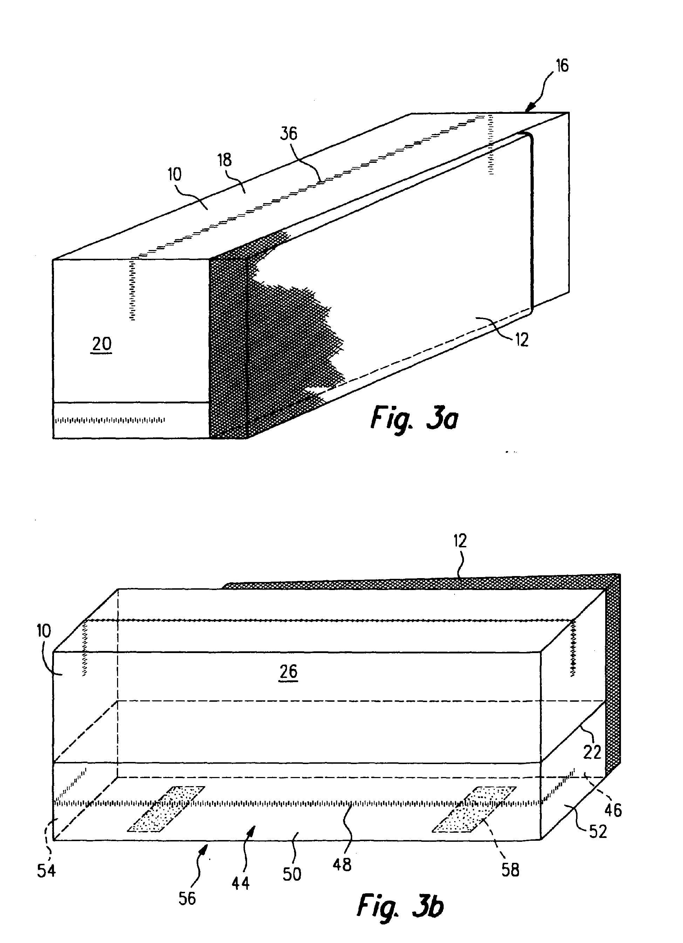

Figuren 3 zeigt nun in den Darstellungen Figur 3a und Figur 3b ein weiteres Erste-Hilfe-Materialbehältnis in zwei Ansichten. Dabei zeigt die Ansicht Figur 3a im Wesentlichen eine Betrachtung von Seiten des zweiten Kompartements 12 aus, wohingegen Figur 3b eine Betrachtung von Seiten des ersten Kompartements 10 aus zeigt. Das zweite Kompartement 12 ist hierbei wie in Figur 2 taschenförmig gestaltet. Das erste Kompartement weist dabei an seinen Seiten 16, 18 sowie 20 einen Reißverschluss auf, der jedoch die Seiten 16 und 20 nicht vollständig übergreift. Der Reißverschluss 36 verläuft vielmehr ausgehend von ca. der Mitte der Seite 16 über die Seite 36 hinsichtlich deren Längserstreckung bis ca. zur Mitte der Seite 20.

Neben dem ersten Kompartement und dem zweiten Kompartement ist darüber hinaus ein drittes Kompartement 44 vorgesehen, dessen Boden 46 ebenfalls wie der Boden 24 mit dem Boden des zweiten Kompartements 12 zusammenfällt und dessen eine Seitenfläche mit der Seitenfläche 22 des ersten Kompartements korrespondiert. Das Volumen des dritten Kompartements 44 entspricht dabei einer Größe, die notwendig ist, um insbesondere ein Warndreieck aufzunehmen. Das dritte Kompartement 44 kann dabei ebenfalls über einen Reißverschluss 48 geöffnet werden, der drei Seiten, nämlich die Deckelfläche 50 sowie die Seitenflächen 52 und 54 übergreift. Die drei Kompartements 10, 12, 44 bilden dabei eine kompakte Einheit.

An der Seitenfläche 56 des dritten Kompartements 44, die insbesondere einer Standfläche entsprechen kann, sind Klettverschlüsse 58 vorgesehen, mit denen das gesamte Behältnis beispielsweise in einem Kraftfahrzeug festgelegt werden kann. Die Seite 56 weist dabei als einzige Seitenfläche keine Öffnungen zum Öffnen und Schließen eines der drei Kompartements 10, 12, 44 auf.

Die Klettverschlüsse 58 erstrecken sich hier lediglich im Bereich der Seite 56 des dritten Kompartements 44.

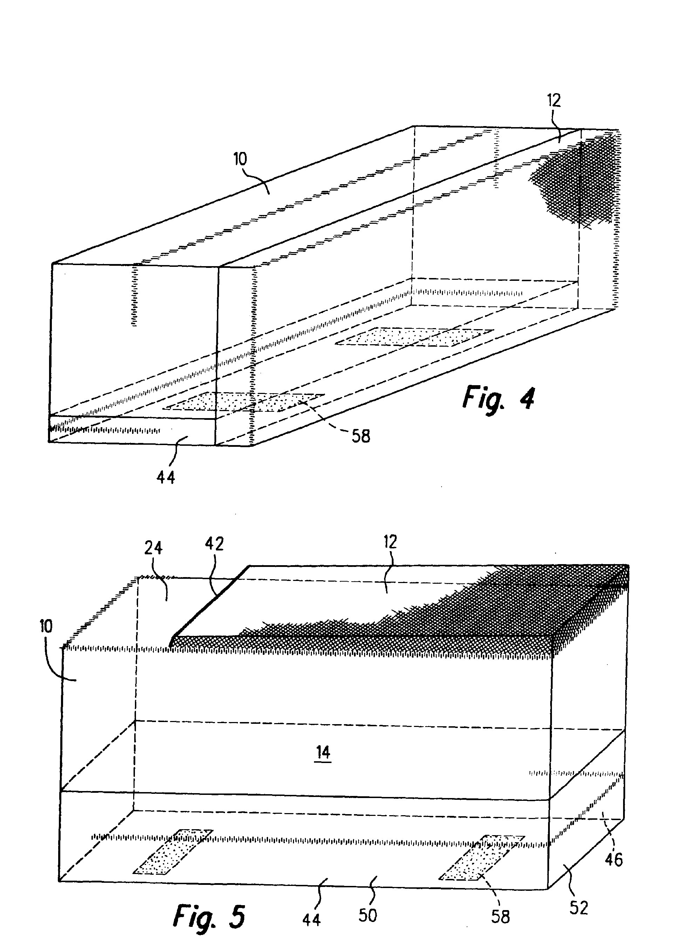

Figur 4 zeigt nun eine abgewandelte Gestaltung zu Figur 3. Die Gestaltung entspricht hinsichtlich des ersten Kompartements 10 sowie des dritten Kompartements 44 der Ausgestaltung gemäß Figur 3. Das zweite Kompartement 12 entspricht dabei im Wesentlichen der Ausgestaltung gemäß Figur 1, wobei hier jedoch vorgesehen ist, bei Figur 4, dass die Klettelemente 58 sich ebenfalls lediglich im Bereich des dritten Kompartements 44 erstrecken.

Es können hierbei insbesondere, wie in Figur 3, zwei Klettverschlüsse vorgesehen sein, um eine sichere Fixierung zu gewährleisten.

Figur 5 zeigt nun eine weitere Ausgestaltung, bei der das zweite Kompartement 12 wiederum taschenmäßig ausgestaltet ist. Anders als bei den Figuren 3 und 4 ist hier jedoch das dritte Kompartement 44 mit der Deckelfläche 14 des ersten Kompartements 10 verbunden. D. h. die Deckelfläche 14 des ersten Kompartements 10 bildet gleichzeitig den Boden des dritten Kompartements 44. Das dritte Kompartement 44 weist wiederum einen Reißverschluss an seinen Seitenflächen 50, 52 sowie an der Seitenfläche 46 auf, so dass ein Aufklappen ermöglicht wird.

Das erste Kompartement weist an seiner Kante zwischen Boden 24 und seinen Seitenwänden einen Reißverschluss auf, der drei von vier Seiten umschließt und bis in die vierte Seite hineinreicht. Auf diese Weise bildet die vierte Seite ein Scharnier zum Aufklappen, wobei die Bodenfläche 24 dann als Bodenfläche des zweiten Kompartements 12 verbleibt. Das zweite Kompartement 12 weist wiederum ein Gummi 42 zur Sicherung der eingeschobenen Schutzbekleidung auf. Ebenfalls sind Klettverschlüsse 58, wie schon beschrieben, vorgesehen, um das Gesamtbehältnis festzulegen.

Grundsätzlich sind eine Vielzahl von Kombinationen der drei Kompartements denkbar, die erfindungsgemäß mit umfasst sein sollen.

## Patentansprüche

1. Erste-Hilfe-Materialbehältnis umfassend eine erstes Kompartement (10) zur Aufnahme von Erste-Hilfe-Material sowie mindestens ein weiteres Kompartement (12, 44), wobei mindestens ein Kompartement (10, 12, 44) öffen- und schließbar ist, **dadurch gekennzeichnet, dass** mindestens eines der weiteren Kompartements (12) zur Aufnahme einer Schutzbekleidung dient und mindestens eine der Wandungen (26) des Kompartements (12) zur Aufnahme der Schutzbekleidung eine Sichtseite bildet und so gestaltet ist, dass die Schutzbekleidung von außen sichtbar ist.

2. Erste-Hilfe-Materialbehältnis nach Anspruch 1, **dadurch gekennzeichnet, dass** ein drittes (44) und/oder viertes Kompartement zur Aufnahme eines oder mehrerer Warndreiecke vorgesehen ist.

3. Erste-Hilfe-Materialbehältnis nach Anspruch 2, **dadurch gekennzeichnet, dass** zwei Warndreiecke vorgesehen sind, die in separaten Kompartements untergebracht sind.

4. Erste-Hilfe-Materialbehältnis nach Anspruch 1 oder 2, **dadurch gekennzeichnet, dass** das Behältnis zumindest teilweise aus einem nicht formstabilen und insbesondere einem textilen Material besteht.

5. Erste-Hilfe-Materialbehältnis nach einem der vorangehenden Ansprüche, **dadurch gekennzeichnet, dass** die Kompartements (10, 12, 44) zumindest zum Teil unabhängig voneinander öffen- und schließbar sind.

6. Erste-Hilfe-Materialbehältnis nach einem der vorangehenden Ansprüche, **dadurch gekennzeichnet, dass** als Mittel zum Öffnen und Schließen der Kompartements Reißverschlüsse (36, 38, 48) vorgesehen sind.

7. Erste-Hilfe-Materialbehältnis nach einem der vorangehenden Ansprüche, **dadurch gekennzeichnet, dass** das Kompartement (12) zur Aufnahme der Schutzbekleidung einseitig oder zweiseitig offen ist, so dass die Schutzbekleidung in das Kompartement (12) einschiebbar ist.

8. Erste-Hilfe-Materialbehältnis nach einem der vorangehenden Ansprüche, **dadurch gekennzeichnet, dass** sich die Kompartements (10, 12, 44) im Wesentlichen eine rechteckige Grundfläche besitzen und jeweils einen Boden, einen Deckel und mindestens drei Seitenwände aufweisen.

9. Erste-Hilfe-Materialbehältnis nach Anspruch 8, **dadurch gekennzeichnet, dass** der Reißverschluss (36, 38, 48) im Bereich der Seitenwände oder zwischen den Seitenwänden und dem Deckel bzw. Boden angeordnet ist.

10. Erste-Hilfe-Materialbehältnis nach Anspruch 8 oder 9, **dadurch gekennzeichnet, dass** der Reißverschluss drei Seiten überspannt, so dass die verbleibende Seite als Scharnier wirkt.

11. Erste-Hilfe-Materialbehältnis nach einem der vorangehenden Ansprüche, **dadurch gekennzeichnet, dass** das Kompartement (12) zur Aufnahme der Schutzbekleidung eine Boden- und eine Deckelfläche umfasst, die zumindest an zwei Seiten unmittelbar miteinander verbunden sind.

12. Erste-Hilfe-Materialbehältnis nach einem der vorangehenden Ansprüche, **dadurch gekennzeichnet, dass** die Sichtseite durch eine Netzmaterial gebildet ist.

13. Erste-Hilfe-Materialbehältnis nach Anspruch 12, **dadurch gekennzeichnet, dass** das Netzmaterial ein Gestricke, Gewirke oder Gewebe ist.

14. Erste-Hilfe-Materialbehältnis nach Anspruch 12 oder 13, **dadurch gekennzeichnet, dass** das Netzmaterial aus insbesondere transluzenten Nylonfäden besteht.

15. Erste-Hilfe-Materialbehältnis nach einem der Ansprüche 12 bis 14, **dadurch gekennzeichnet, dass** der vom Netz abgedeckte Anteil der Sichtseite zwischen 30 und 70%, vorzugsweise zwischen 35 und 50% und vorzugsweise zwischen 40 und 45% liegt.

16. Erste-Hilfe-Materialbehältnis nach einem der vorangehenden Ansprüche, **dadurch gekennzeichnet, dass** mindestens zwei der Kompartements (10, 12, 44) über ihre Böden (24) bzw. Deckel (14) miteinander verbunden sind, insbesondere die jeweiligen Kompartements (10, 12, 44) einen gemeinsamen Boden/Deckel (12, 24) aufweisen.

17. Erste-Hilfe-Materialbehältnis nach einem der vorangehenden Ansprüche, **dadurch gekennzeichnet, dass** die Sichtseite mit einer Boden- und /oder Deckelfläche (26) korrespondiert und die Boden- und/oder Deckelfläche (26) ganz oder teilweise als Sichtseite ausgebildet ist.

18. Erste-Hilfe-Materialbehältnis nach einem der vorangehenden Ansprüche, **dadurch gekennzeichnet, dass** die Kompartements (10, 12, 44) an die Form und Größe der aufzunehmenden Gegenstände angepasst sind.

19. Erste-Hilfe-Materialbehältnis nach einem der vorangehenden Ansprüche, **dadurch gekennzeichnet, dass** mindestens zwei Kompartements (10, 12, 44) die gleiche Grundfläche aufweisen.
